# EUROPÄISCHE PATENTSCHRIFT

(11) **EP 3 117 968 B1**
(45) Veröffentlichungstag und Bekanntmachung des Hinweises auf die Patenterteilung: **11.11.2020**
(21) Anmeldenummer: 16001270.4
(22) Anmeldetag: 06.06.2016
(51) Int. Cl.: B25J 9/16, G05B 19/423

(54) **STEUERN EINES NACHGIEBIG GEREGELTEN ROBOTERS**
CONTROL OF A FLEXIBLY CONTROLLED ROBOT
COMMANDE D'UN ROBOT REGLÉ DE FAÇON ÉLASTIQUE

(30) Priorität: 13.07.2015 DE 102015009048
(43) Veröffentlichungstag der Anmeldung: 18.01.2017
(73) Patentinhaber: KUKA Deutschland GmbH, 86165 Augsburg (DE)
(72) Erfinder: Rohmer, Matthias, 86157 Augsburg (DE); Reichl, Tobias, 80939 München (DE); Schreiber, Günter, 86316 Friedberg (DE); Mönnich, Holger, 86316 Friedberg (DE); Mueller-Sommer, Martin, 86447 Aindling (DE); Bonin, Uwe, 86161 Augsburg (DE)
(74) Vertreter: Schlotter, Alexander Carolus Paul

(56) Entgegenhaltungen:
- US-A1- 2004 128 026
- US-A1- 2006 142 657
- US-A1- 2012 158 011
- HO S C ET AL: "ROBOT ASSISTED KNEE SURGERY. ÖESTABLISHING A FORCE CONTROL STRATEGY INCORPORATING ACTIVE MOTION CONSTRAINT", IEEE ENGINEERING IN MEDICINE AND BIOLOGY MAGAZINE, IEEE SERVICE CENTER, PISACATAWAY, NJ, US, Bd. 14, Nr. 3, 1. Mai 1995 (1995-05-01), Seiten 292-300, XP000505085, ISSN: 0739-5175, DOI: 10.1109/51.391774

## Beschreibung

Die vorliegende Erfindung betrifft ein Verfahren und eine Steuerung zum Steuern eines nachgiebig geregelten Roboters sowie ein Computerprogrammprodukt zur Durchführung des Verfahrens und eine Roboteranordnung mit der Steuerung.

Aus der US 2004/0128026 A1 ist ein Verfahren zum Steuern eines nachgiebig geregelten Roboters bekannt, wobei in einem ersten, freien Bereich eine Admittanz des Roboters, mit der dieser einer Betätigungskraft eines Chirurgen folgt, hoch und eine Steifigkeit und Dämpfung, mit der der Roboter steuerungstechnisch an seine aktuelle Position gefesselt ist, niedrig ist.

In einem dritten, gesperrten Bereich ist die Steifigkeit und Dämpfung, mit der der Roboter steuerungstechnisch an eine seiner aktuellen Position nächste Position auf der Grenze des gesperrten Bereichs gefesselt ist, sehr hoch, um den Roboter aus dem gesperrten Bereich zurückzutreiben.

In einem zweiten Bereich zwischen dem ersten und dritten Bereich wird die Admittanz reduziert und die Steifigkeit und Dämpfung, mit der der Roboter steuerungstechnisch an seine aktuelle Position gefesselt ist, erhöht.

Wird eine solche Grenzüberwachung aktiviert, während der Roboter sich bereits weit von der Grenze entfernt innerhalb des gesperrten Bereichs befindet, ergibt sich unmittelbar eine massive Rückstellbewegung des Roboters, die zu einem unerwarteten und/oder -wünschten Verhalten führen kann.

HO S C ET AL: "ROBOT ASSISTED KNEE SURGERY. ÖESTABLISHING A FORCE CONTROL STRATEGY INCORPORATING ACTIVE MOTION CONSTRAINT", IEEE ENGINEERING IN MEDICINE AND BIOLOGY MAGAZINE, Bd: 14, Nr:3, Seiten 292 - 300, 1. Mai 1995, XP000505085 beschreibt eine Regelungsstrategie für einen robotergeführten Knochenfräser, bei dem eine Widerstandskraft, die einer durch einen Chirurgen geführten Bewegung von einer Grenze weg weiter in einen hinein Sperrbereich entgegenwirkt, konstant hoch ist.

Aufgabe der vorliegenden Erfindung ist es, den Betrieb eines nachgiebig geregelten Roboters zu verbessern.

Diese Aufgabe wird durch ein Verfahren mit den Merkmalen des Anspruchs 1 gelöst. Ansprüche 9 bis 11 stellen eine Steuerung bzw. ein Computerprogrammprodukt zur Durchführung eines hier beschriebenen Verfahrens bzw. eine Roboteranordnung mit einer hier beschriebenen Steuerung unter Schutz. Die Unteransprüche betreffen vorteilhafte Weiterbildungen.

Nach einem Aspekt der vorliegenden Erfindung umfasst ein Verfahren zum Steuern eines nachgiebig geregelten Roboters den Schritt: Durchführen einer Grenzüberwachung des Roboters, wobei eine erste Rückstellkraft, die den Roboter aus einer aktuellen Position in einem gesperrten Bereich zu einer Grenze dieses Bereichs zurücktreibt bzw. zurücktreiben sucht, steuerungstechnisch vorgegeben bzw. kommandiert, insbesondere aufgeprägt wird, sofern bzw. in Abhängigkeit davon, ob der Roboter sich bei Aktivierung der Grenzüberwachung bereits in dieser Position in dem gesperrten Bereich befindet, und eine zweite Rückstellkraft, die den Roboter aus der(selben) Position zu der Grenze zurücktreibt bzw. zurücktreiben sucht, steuerungstechnisch vorgegeben bzw. kommandiert, insbesondere aufgeprägt wird, sofern bzw. in Abhängigkeit davon, ob der Roboter erst nach Aktivierung der Grenzüberwachung diese Position in dem gesperrten Bereich einnimmt bzw. aufweist, wobei die erste Rückstellkraft stets oder wenigstens zeitweise, insbesondere wenigstens im Anschluss an eine Aktivierung der Grenzüberwachung, kleiner ist als die zweite Rückstellkraft, insbesondere stets oder wenigstens zeitweise, insbesondere wenigstens im Anschluss an eine Aktivierung der Grenzüberwachung gleich Null ist und/oder stets oder wenigstens zeitweise, insbesondere wenigstens im Anschluss an eine Aktivierung der Grenzüberwachung höchstens 75%, insbesondere höchstens 50%, insbesondere höchstens 25% der zweiten Rückstellkraft beträgt.

Eine Steuerung nach einem Aspekt der vorliegenden Erfindung ist zur Durchführung eines hier beschriebenen Verfahrens, insbesondere hard- und/oder softwaretechnisch, eingerichtet und/oder umfasst Mittel zum Durchführen einer Grenzüberwachung des Roboters, Mittel zum steuerungstechnischen Vorgeben bzw. Kommandieren, insbesondere Aufprägen, einer ersten Rückstellkraft, die den Roboter aus einer aktuellen Position in einem gesperrten Bereich zu einer Grenze dieses Bereichs zurücktreibt bzw. zurücktreiben sucht, sofern bzw. in Abhängigkeit davon, ob der Roboter sich bei Aktivierung der Grenzüberwachung bereits in dieser Position in dem gesperrten Bereich befindet, und Mittel zum steuerungstechnischen Vorgeben bzw. Kommandieren, insbesondere Aufprägen, einer zweiten Rückstellkraft, die den Roboter aus der(selben) Position zu der Grenze zurücktreibt bzw. zurücktreiben sucht, sofern bzw. in Abhängigkeit davon, ob der Roboter erst nach Aktivierung der Grenzüberwachung diese Position in dem gesperrten Bereich einnimmt bzw. aufweist, wobei die erste Rückstellkraft stets oder wenigstens zeitweise, insbesondere wenigstens im Anschluss an eine Aktivierung der Grenzüberwachung, kleiner ist als die zweite Rückstellkraft, insbesondere stets oder wenigstens zeitweise, insbesondere wenigstens im Anschluss an eine Aktivierung der Grenzüberwachung gleich Null ist und/oder stets oder wenigstens zeitweise, insbesondere wenigstens im Anschluss an eine Aktivierung der Grenzüberwachung höchstens 75%, insbesondere höchstens 50%, insbesondere höchstens 25% der zweiten Rückstellkraft beträgt.

Hierdurch kann vorteilhaft eine unerwartete und/oder -wünschte massive steuerungstechnisch induzierte Rückstellbewegung des Roboters aus dem gesperrten Bereich zu dessen Grenze zurück bei bzw. infolge einer Aktivierung der Grenzüberwachung verhindert oder jedenfalls reduziert werden.

Entsprechend wird in einer Ausführung festgestellt bzw. unterschieden, ob eine aktuelle Position des Roboters in dem gesperrten Bereich bereits bei Aktivierung der Grenzüberwachung vorliegt oder erst nach Aktivierung der Grenzüberwachung eingenommen wird bzw. worden ist, und entsprechend alternativ die erste oder zweite Rückstellkraft vorgegeben bzw. kommandiert, insbesondere steuerungstechnisch aufgeprägt. Entsprechend weist die Steuerung in einer Ausführung Mittel zum Feststellen bzw. Unterscheiden, ob eine aktuelle Position des Roboters in dem gesperrten Bereich bereits bei Aktivierung der Grenzüberwachung vorliegt oder erst nach Aktivierung der Grenzüberwachung eingenommen wird bzw. worden ist, auf.

In einer Ausführung wird eine Steifigkeit einer virtuellen Feder, die den Roboter steuerungstechnisch an eine Ankerposition, insbesondere auf der Grenze, fesselt, vorgegeben, wobei eine erste Steifigkeit vorgegeben, insbesondere realisiert, wird, sofern der Roboter sich bei Aktivierung der Grenzüberwachung bereits (in einer bzw. der Position) in dem gesperrten Bereich befindet, und eine zweite Steifigkeit vorgegeben, insbesondere realisiert, wird, sofern der Roboter erst nach Aktivierung der Grenzüberwachung dieselbe Position in dem gesperrten Bereich einnimmt bzw. aufweist bzw. in den gesperrten Bereich eindringt, wobei die erste Steifigkeit stets oder wenigstens bei Aktivierung der Grenzüberwachung kleiner als die zweite Steifigkeit ist, insbesondere gleich Null ist oder höchstens 75%, insbesondere höchstens 50%, insbesondere höchstens 25% der zweiten Steifigkeit beträgt.

Entsprechend umfasst die Steuerung in einer Ausführung Mittel zum Vorgeben einer Steifigkeit einer virtuellen Feder, die den Roboter steuerungstechnisch an eine Ankerposition, insbesondere auf der Grenze, fesselt, wobei dieses Mittel in einer Ausführung Mittel zum Vorgeben einer ersten Steifigkeit, sofern der Roboter sich bei Aktivierung der Grenzüberwachung bereits (in einer bzw. der Position) in dem gesperrten Bereich befindet, und Mittel zum Vorgeben einer zweiten Steifigkeit aufweist, sofern der Roboter erst nach Aktivierung der Grenzüberwachung dieselbe Position in dem gesperrten Bereich einnimmt bzw. aufweist bzw. in den gesperrten Bereich eindringt, wobei die erste Steifigkeit stets oder wenigstens bei Aktivierung der Grenzüberwachung kleiner als die zweite Steifigkeit ist, insbesondere gleich Null ist oder höchstens 75%, insbesondere höchstens 50%, insbesondere höchstens 25% der zweiten Steifigkeit beträgt.

Zusätzlich oder alternativ wird in einer Ausführung eine Steifigkeit einer bzw. der virtuellen Feder, die den Roboter steuerungstechnisch an eine bzw. die Ankerposition, insbesondere auf der Grenze, fesselt, in Abhängigkeit von einem zeitlichen Abstand zur Aktivierung der Grenzüberwachung vorgegeben, insbesondere realisiert, insbesondere auch oder nur sofern der Roboter sich bei Aktivierung der Grenzüberwachung bereits in dem gesperrten Bereich befindet. In einer Ausführung nimmt die Steifigkeit mit einer Zeit, die seit Aktivierung der Grenzüberwachung verstrichen ist, zu, insbesondere kontinuierlich, insbesondere linear, oder diskontinuierlich bzw. in einem oder mehreren diskreten Sprüngen. Die Steifigkeit nimmt in einer Ausführung mit der Zeit von Null und/oder auf ein Maximum zu.

Entsprechend umfasst in einer Ausführung das Mittel zum Vorgeben einer Steifigkeit einer virtuellen Feder Mittel zum Vorgeben der Steifigkeit in Abhängigkeit von einem zeitlichen Abstand zur Aktivierung der Grenzüberwachung, insbesondere auch oder nur sofern der Roboter sich bei Aktivierung der Grenzüberwachung bereits in dem gesperrten Bereich befindet.

Zusätzlich oder alternativ wird in einer Ausführung eine Steifigkeit einer bzw. der virtuellen Feder, die den Roboter steuerungstechnisch an eine bzw. die Ankerposition, insbesondere auf der Grenze, fesselt, in Abhängigkeit von einer Bewegung des Roboters relativ zur Grenze vorgegeben, insbesondere realisiert, insbesondere (derart, dass sie) bei einer Bewegung von der Grenze weg, insbesondere stets oder wenigstens zeitweise, insbesondere wenigstens im Anschluss an eine Aktivierung der Grenzüberwachung, größer (ist) als bei einer Bewegung nicht von der Grenze weg, insbesondere einer Bewegung zur Grenze hin oder parallel zur Grenze, insbesondere auch oder nur sofern der Roboter sich bei Aktivierung der Grenzüberwachung bereits in dem gesperrten Bereich befindet. In einer Ausführung ist bzw. beträgt eine Steifigkeit bei einer Bewegung nicht von der Grenze weg, insbesondere einer Bewegung zur Grenze hin oder parallel zur Grenze, insbesondere stets oder wenigstens zeitweise, insbesondere wenigstens im Anschluss an eine Aktivierung der Grenzüberwachung, höchstens 75%, insbesondere höchstens 50%, insbesondere höchstens 25% der Steifigkeit bei einer Bewegung von der Grenze weg.

Entsprechend umfasst in einer Ausführung das Mittel zum Vorgeben einer Steifigkeit einer virtuellen Feder Mittel zum Vorgeben der Steifigkeit in Abhängigkeit von einer Bewegung des Roboters relativ zur Grenze, insbesondere (derart, dass sie) bei einer Bewegung von der Grenze weg, insbesondere stets oder wenigstens zeitweise, insbesondere wenigstens im Anschluss an eine Aktivierung der Grenzüberwachung, größer (ist) als bei einer Bewegung nicht von der Grenze weg, insbesondere einer Bewegung zur Grenze hin oder parallel zur Grenze, insbesondere auch oder nur sofern der Roboter sich bei Aktivierung der Grenzüberwachung bereits in dem gesperrten Bereich befindet.

Durch eine solche richtungsabhängige, zeitabhängige und/oder von der aktuellen Position des Roboters bei Aktivierung der Grenzüberwachung abhängige Steifigkeit einer virtuellen, steuerungstechnisch realisierten Feder kann vorteilhaft die erste bzw. zweite Rückstellkraft vorgegeben bzw. kommandiert, insbesondere aufgeprägt werden.

In einer Ausführung umfasst ein Verfahren zum Steuern des nachgiebig geregelten Roboters den Schritt: Durchführen einer bzw. der Grenzüberwachung des Roboters, wobei eine bzw. die erste Rückstellkraft, die den Roboter aus einer bzw. der aktuellen Position in einem bzw. dem gesperrten Bereich zu einer bzw. der Grenze dieses Bereichs zurücktreibt bzw. zurücktreiben sucht, von einem Abstand der Position zu der Grenze unabhängig, insbesondere gleich Null, steuerungstechnisch vorgegeben bzw. kommandiert, insbesondere aufgeprägt wird, sofern der Roboter um einen Weg zu der Grenze hin oder parallel zu der Grenze hin bewegt wird, und eine andere, insbesondere erste, Rückstellkraft, die größer als die erste Rückstellkraft ist, insbesondere wenigstens 125%, insbesondere wenigstens 150%, insbesondere wenigstens 200% der ersten Rückstellkraft beträgt, steuerungstechnisch vorgegeben bzw. kommandiert, insbesondere aufgeprägt wird, sofern der Roboter um denselben Weg von der Grenze weg bewegt wird, insbesondere auch oder nur sofern der Roboter sich bei Aktivierung der Grenzüberwachung bereits in (dieser Position in) dem gesperrten Bereich befindet, insbesondere stets oder wenigstens zeitweise, insbesondere wenigstens im Anschluss an eine Aktivierung der Grenzüberwachung.

Die Steuerung umfasst entsprechend in einer Ausführung Mittel zum Durchführen einer bzw. der Grenzüberwachung des Roboters, Mittel zum steuerungstechnischen Vorgeben bzw. Kommandieren, insbesondere Aufprägen, einer bzw. der ersten Rückstellkraft, die den Roboter aus einer bzw. der aktuellen Position in einem bzw. dem gesperrten Bereich zu einer bzw. der Grenze dieses Bereichs zurücktreibt bzw. zurücktreiben sucht, unabhängig von einem Abstand der Position zu der Grenze, insbesondere gleich Null, sofern der Roboter um einen Weg zu der Grenze hin oder parallel zu der Grenze hin bewegt wird, und Mittel zum steuerungstechnischen Vorgeben bzw. Kommandieren, insbesondere Aufprägen, einer anderen, insbesondere ersten, Rückstellkraft, die größer als die erste Rückstellkraft ist, insbesondere wenigstens 125%, insbesondere wenigstens 150%, insbesondere wenigstens 200% der ersten Rückstellkraft beträgt, sofern der Roboter um denselben Weg von der Grenze weg bewegt wird, insbesondere auch oder nur sofern der Roboter sich bei Aktivierung der Grenzüberwachung bereits in (dieser Position in) dem gesperrten Bereich befindet, insbesondere stets oder wenigstens zeitweise, insbesondere wenigstens im Anschluss an eine Aktivierung der Grenzüberwachung.

Mit anderen Worten wird nach diesem Aspekt einer, insbesondere handgeführten, Bewegung des nachgiebig geregelten Roboters zu der Grenze hin oder parallel zu der Grenze steuerungstechnisch ein kleinerer Widerstand, insbesondere kein Widerstand, entgegengesetzt als einer Bewegung (weiter) von der Grenze weg in den gesperrten Bereich hinein. Hierdurch kann insbesondere vorteilhaft bei Aktivierung einer Grenzüberwachung eine relativ kleine, insbesondere auch verschwindende Rückstellkraft, zur Grenze hin aufgeprägt und so eine unerwartete und/oder -wünschte massive steuerungstechnisch induzierte Rückstellbewegung des Roboters aus dem gesperrten Bereich zu dessen Grenze zurück bei bzw. infolge einer Aktivierung der Grenzüberwachung verhindert oder jedenfalls reduziert und zugleich eine unerwünschte weitere Bewegung in den gesperrten Bereich hinein wirkungsvoll verhindert oder jedenfalls erschwert werden.

In einer Ausführung ist hierzu eine Ankerposition einer bzw. der virtuellen Feder, an die der Roboter steuerungstechnisch gefesselt ist, durch eine externe Kraftbeaufschlagung, insbesondere Handführung, des Roboters, wenigstens im Wesentlichen, nicht von der Grenze weg verschiebbar. Zusätzlich oder alternativ wird in einer Ausführung als eine bzw. die Ankerposition einer bzw. der virtuellen Feder, an die der Roboter steuerungstechnisch gefesselt ist, eine bzw. die aktuelle Position des Roboters oder eine Position innerhalb einer, insbesondere kürzesten, Verbindung zwischen der aktuellen Position und der Grenze vorgegeben, die um eine, insbesondere fest vorgegebene oder variabel vorgebbare, Distanz von der aktuellen Position zu der Grenze hin beabstandet ist.

Entsprechend umfasst in einer Ausführung die Steuerung Mittel zum Vorgeben einer bzw. der aktuellen Position des Roboters oder einer Position innerhalb einer, insbesondere kürzesten, Verbindung zwischen der aktuellen Position und der Grenze, die um eine, insbesondere fest vorgegebene oder variabel vorgebbare, Distanz von der aktuellen Position zu der Grenze hin beabstandet ist, als eine bzw. die Ankerposition einer bzw. der virtuellen Feder, an die der Roboter steuerungstechnisch gefesselt ist.

Mit anderen Worten wird in dieser Ausführung eine Ankerposition auf eine aktuelle Position oder auf eine um eine Distanz von dieser zu der Grenze hin beabstandete Position gesetzt bzw. mit der aktuellen Position mitgeführt, sofern die aktuelle Position nicht von der Grenze weg bewegt wird, insbesondere sofern die aktuelle Position zur Grenze hin oder parallel zur Grenze bewegt wird, und die bisherige Ankerposition beibehalten, sofern der Roboter von der Grenze weg bewegt wird.

Die Distanz ist in einer Ausführung kürzer als der (kürzeste) Abstand der aktuellen Position zu der Grenze.

In einer Ausführung wird eine Dämpfungskraft, die einer Bewegung des Roboters entgegenwirkt und von einer aktuellen Geschwindigkeit des Roboters abhängt, steuerungstechnisch derart vorgegeben bzw. kommandiert, insbesondere aufgeprägt, dass sie in einem zweiten Geschwindigkeitsbereich oberhalb einer vorgegebenen Mindestgeschwindigkeit stärker mit der aktuellen Geschwindigkeit des Roboters ansteigt als in einem ersten Geschwindigkeitsbereich unterhalb der Mindestgeschwindigkeit.

Ein Mittel im Sinne der vorliegenden Erfindung kann hard- und/oder softwaretechnisch ausgebildet sein, insbesondere eine, vorzugsweise mit einem Speicher- und/oder Bussystem daten- bzw. signalverbundene, insbesondere digitale, Verarbeitungs-, insbesondere Mikroprozessoreinheit (CPU) und/oder ein oder mehrere Programme oder Programmmodule aufweisen. Die CPU kann dazu ausgebildet sein, Befehle, die als ein in einem Speichersystem abgelegtes Programm implementiert sind, abzuarbeiten, Eingangssignale von einem Datenbus zu erfassen und/oder Ausgangssignale an einen Datenbus abzugeben. Ein Speichersystem kann ein oder mehrere, insbesondere verschiedene, Speichermedien, insbesondere optische, magnetische, Festkörper- und/oder andere nicht-flüchtige Medien aufweisen. Das Programm kann derart beschaffen sein, dass es die hier beschriebenen Verfahren verkörpert bzw. auszuführen imstande ist, sodass die CPU die Schritte solcher Verfahren ausführen kann und damit insbesondere den Roboter steuern kann.

Der nachgiebig geregelte Roboter ist in einer Ausführung impedanz- oder admittanzgeregelt, insbesondere gravitations- und/oder reibungskompensiert geregelt, beispielsweise wie in der eingangs genannten US 2004/0128026 A1 beschrieben, auf die diesbezüglich ergänzend vollinhaltlich Bezug genommen wird. Insbesondere wird unter einem nachgiebig geregelten Roboter ein Roboter verstanden, der bzw. dessen Steuerung eine auf den Roboter, insbesondere durch eine Handführung bzw. manuell, aufgeprägte externe Kraft erfasst und eine Bewegung (des Roboters) in Abhängigkeit von dieser Kraft kommandiert bzw. ausführt, insbesondere in Abhängigkeit von Größe und/oder Richtung der Kraft, bzw. hierzu hard- und/oder softwaretechnisch eingerichtet ist.

Die externe Kraft kann insbesondere auf Basis von Kräften in den Gelenken und/oder Antrieben des Roboters oder mittels eines Kraftsensors an einem Handgriff des Roboters erfasst werden.

Der gesperrte Bereich bzw. seine Grenze kann insbesondere im Gelenkkoordinatenraum des Roboters definiert bzw. vorgegeben werden bzw. sein, insbesondere in Form von steuerungstechnischen Achsanschlägen bzw. -begrenzungen. Gleichermaßen kann der gesperrte Bereich bzw. seine Grenze insbesondere im kartesischen bzw. Arbeitsraum des Roboters vorgegeben sein, insbesondere in Form von Begrenzungen bzw. Anschlägen seines TCPs oder einer anderen roboterfesten Referenz. Entsprechend kann eine (aktuelle) Position bzw. Geschwindigkeit des Roboters insbesondere im Arbeits- oder Gelenkkoordinatenraum des Roboters definiert sein und somit insbesondere die Position bzw. Geschwindigkeit des TCPs oder einer anderen roboterfesten Referenz oder einer oder mehrerer Achsen des Roboters angeben bzw. sein.

Zur kompakteren Darstellung wird vorliegend auch ein antiparalleles Kräftepaar bzw. ein Drehmoment verallgemeinernd als eine Kraft bezeichnet.

Unter einer Grenzüberwachung wird vorliegend insbesondere eine Überwachung verstanden, ob eine aktuelle Position des Roboters innerhalb eines gesperrten Bereichs liegt oder nicht.

In einer Ausführung werden ein oder mehrere, insbesondere alle, Schritte des Verfahrens teilweise oder vollständig automatisiert, insbesondere durch die Steuerung, durchgeführt.

Weitere Vorteile und Merkmale ergeben sich aus den Unteransprüchen und den Ausführungsbeispielen. Hierzu zeigt, teilweise schematisiert:
- Fig. 1:: ein Verfahren zum Steuern eines nachgiebig geregelten Roboters nach einer Ausführung der vorliegenden Erfindung;
- Fig. 2:: ein Aufprägen einer Rückstellkraft in einem gesperrten Bereich nach einer Ausführung der vorliegenden Erfindung;
- Fig. 3:: ein Aufprägen einer Rückstellkraft in einem gesperrten Bereich nach einer weiteren Ausführung der vorliegenden Erfindung; und
- Fig. 4:: eine Roboteranordnung mit dem Roboter und einer Steuerung nach einer Ausführung der vorliegenden Erfindung.

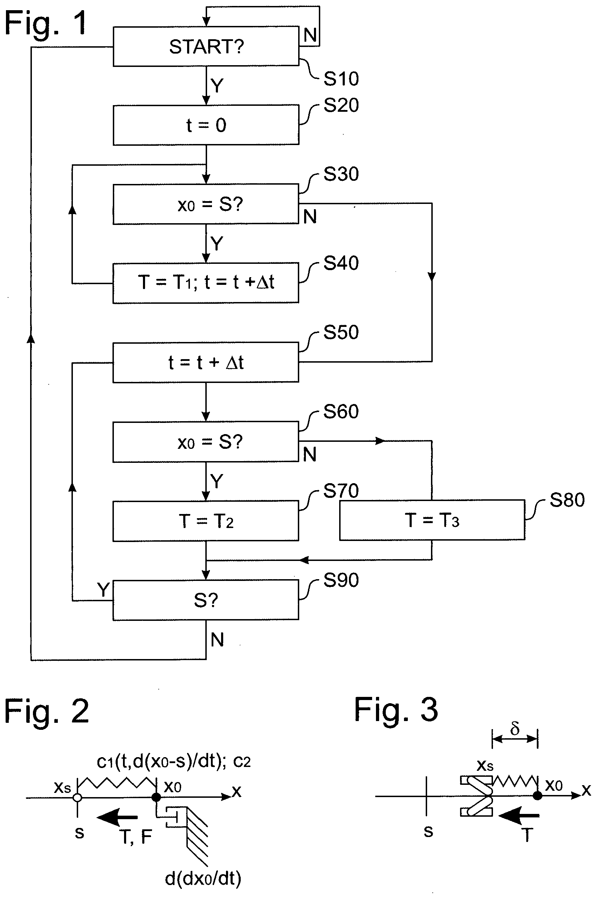

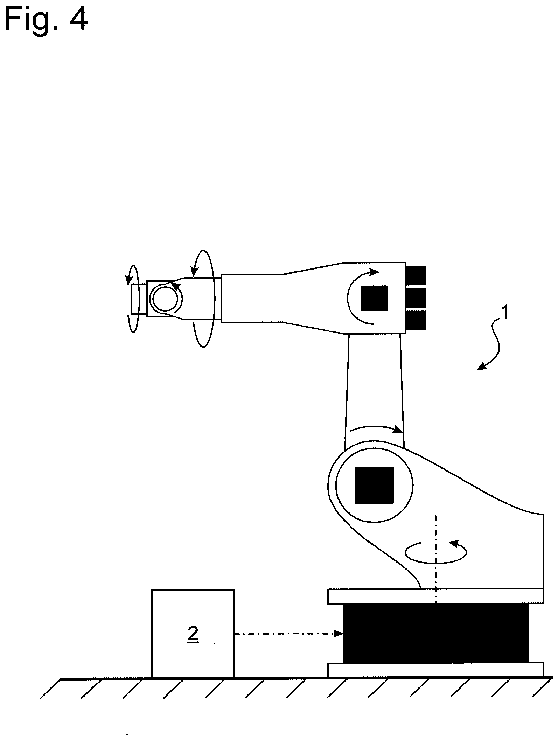

Fig. 4 zeigt eine Roboteranordnung mit einer Steuerung 2 und einem durch diese nachgiebig geregelten mehrachsigen Roboter 1 nach einer Ausführung der vorliegenden Erfindung. Die Steuerung 2 führt ein nachfolgend mit Bezug auf Fig. 1-3 erläutertes Verfahren zum Steuern des Roboters 1 aus bzw. ist hierzu hard- und softwaretechnisch eingerichtet.

In einem ersten Schritt S10 stellt die Steuerung 2 fest, ob eine Grenzüberwachung des Roboters aktiviert worden ist. Solange dies nicht der Fall ist (S10: "N"), wird Schritt S10 wiederholt.

Stellt die Steuerung 2 fest, dass eine Grenzüberwachung des Roboters aktiviert worden ist (S10: "Y"), wird in Schritt S20 ein Zeitzähler t initialisiert und in Schritt S30 geprüft, ob eine aktuelle Position x₀ des Roboters 1 bereits bei Aktivierung der Grenzüberwachung in einem gesperrten Bereich S liegt.

Die Position x kann eine ein- oder mehrdimensionale Position sein und beispielsweise die Stellung einer oder mehrerer Achsen des Roboters 1 oder die Lage und/oder Position seines TCPs im Arbeitsraum darstellen bzw. sein. In den Fig. 2, 3 ist sie zur besseren Übersichtlichkeit eindimensional dargestellt, wobei s die Grenze und x > s den gesperrten Bereich S darstellen.

Stellt die Steuerung 2 in Schritt S30 fest, dass die aktuelle Position x₀ des Roboters 1 bereits bei Aktivierung der Grenzüberwachung im gesperrten Bereich S liegt (S30: "Y"), fährt sie mit Schritt S40 fort, andernfalls überspringt sie diesen und fährt mit Schritt S50 fort.

In Schritt S40 gibt die Steuerung 2 steuerungstechnisch eine erste Rückstellkraft T₁ vor, die den Roboter 1 aus seiner aktuellen Position x₀ in dem gesperrten Bereich S zu der Grenze s dieses Bereichs zurücktreibt.

Außerdem wird in Schritt S40 der Zeitzähler t inkrementiert, anschließend kehrt die Steuerung bzw. das Verfahren zu Schritt S30 zurück. Somit gibt die Steuerung 2 steuerungstechnisch die erste Rückstellkraft T₁ vor, bis die aktuelle Position x₀ nicht mehr in dem gesperrten Bereich S liegt (S30: "N"), und fährt dann mit Schritt S50 fort.

In Schritt S50 wird der Zeitzähler t inkrementiert und in einem darauffolgenden Schritt S60 geprüft, ob die aktuelle Position x₀ des Roboters 1 (nun) in dem gesperrten Bereich S liegt. Somit wird - im Ausführungsbeispiel durch die Inkrementierung S50 angedeutet - in Schritt S60 festgestellt, ob der Roboter 1 eine aktuelle Position x₀ in dem gesperrten Bereich S erst nach Aktivierung der Grenzüberwachung einnimmt.

Stellt die Steuerung 2 in Schritt S60 fest, dass der Roboter 1 - erst nach Aktivierung der Grenzüberwachung (vgl. S50) - eine aktuelle Position x₀ im gesperrten Bereich S einnimmt (S60: "Y"), fährt sie mit Schritt S70 fort, andernfalls, d.h. wenn die aktuelle Position x₀ nicht im gesperrten Bereich S liegt (S60: "N"), fährt sie mit Schritt S80 fort.

In Schritt S70 gibt die Steuerung 2 steuerungstechnisch eine zweite Rückstellkraft T₂ vor, die den Roboter 1 ebenfalls aus seiner aktuellen Position x₀ in dem gesperrten Bereich S zu der Grenze s dieses Bereichs zurücktreibt.

In Schritt S80 wird der Roboter 1 hingegen beispielsweise wie in der US 2004/0128026 A1 beschrieben nachgiebig geregelt, indem eine (mehrdimensionale) Antriebskraft T₃ in Abhängigkeit von einer manuell aufgeprägten externen Kraft zur Handführung des Roboters und eines (positiven) Abstandes zur Grenze s kommandiert wird.

Im Anschluss an Schritt S70 und S80 wird in Schritt S90 jeweils geprüft, ob die Grenzüberwachung weiterhin aktiviert ist und in diesem Falle (S90: "Y") mit Schritt S50 fortgefahren, andernfalls, d.h. bei Deaktivierung der Grenzüberwachung (S90: "N") mit Schritt S10.

Fig. 2 veranschaulicht eine Impedanz- bzw. Admittanzregelung des nachgiebig geregelten Roboters 1 in dem gesperrten Bereich x>s. Hierbei ist der Roboter 1 bzw. seine aktuelle Position x₀ durch eine virtuelle Feder steuerungstechnisch an eine Ankerposition xₛ auf der Grenze s und durch einen virtuellen Dämpfer steuerungstechnisch inertial bzw. an die Umgebung gefesselt.

Sofern der Roboter sich bei Aktivierung der Grenzüberwachung bereits in der aktuelle Position x₀ in dem gesperrten Bereich x>s befindet (S30: "Y"), wird eine erste Steifigkeit c₁ der Feder vorgegeben.

Sofern der Roboter hingegen erst nach Aktivierung der Grenzüberwachung (vgl. S50: t + Δt) dieselbe Position x₀ in dem gesperrten Bereich x>s einnimmt (S60: "Y"), wird eine zweite Steifigkeit c₂ der Feder vorgegeben.

Die erste Steifigkeit c₁ ist im Ausführungsbeispiel zunächst gleich Null und wächst anschließend in Abhängigkeit von einem zeitlichen Abstand t zur Aktivierung t= 0 der Grenzüberwachung (vgl. S20) bis auf den Wert der zweiten Steifigkeit c₂.

Auf diese Weise ist die erste Steifigkeit c₁ bei Aktivierung der Grenzüberwachung und im Anschluss daran zunächst kleiner als die zweite Steifigkeit c₂. In einer Abwandlung kann die erste Steifigkeit c₁ auch dauerhaft kleiner als die zweite Steifigkeit c₂ sein.

Entsprechend ist in derselben aktuellen Position x₀ im gesperrten Bereich die erste Rückstellkraft T₁ = c₁·(x₀-s), die durch die virtuelle Feder steuerungstechnisch aufgeprägt wird, wenigstens zeitweise kleiner als die zweite Rückstellkraft T₂ = c₂·(x₀-s), insbesondere wenigstens zunächst gleich Null.

Hierdurch wird vorteilhaft eine überraschende massive Rückstellbewegung des Roboters 1 infolge einer Aktivierung der Grenzüberwachung in einer aktuellen Position im gesperrten Bereich verhindert.

Die Steifigkeit c₁ der virtuellen Feder, die den Roboter steuerungstechnisch an die Ankerposition xₛ auf der Grenze s fesselt, wird bei einer Bewegung von der Grenze s weg größer vorgegeben als bei einer Bewegung zu der Grenze hin oder parallel zu dieser: c₁(d(x₀-s)/dt > 0) > c₁(d(x₀-s)/dt < 0). Mit anderen Worten wird einer handgeführten Bewegung des Roboters weiter in den gesperrten Bereich hinein (d(x₀-s)/dt > 0) ein stärkerer Widerstand entgegengesetzt. Insbesondere kann die Steifigkeit c₁ für eine Bewegung zu der Grenze hin oder parallel zu dieser wenigstens anfänglich gleich Null sein, so dass (zunächst) keine selbsttätige Rückstellung eingeleitet wird, während die Steifigkeit c₁ für eine Bewegung von der Grenze weg bereits anfänglich größer Null sein kann, so dass dieser bereits von Anfang steuerungstechnisch an ein Widerstand entgegengesetzt wird.

Fig. 3 veranschaulicht eine Impedanz- bzw. Admittanzregelung des nachgiebig geregelten Roboters 1 in dem gesperrten Bereich x>s nach einer weiteren Ausführung der vorliegenden Erfindung. Hierbei ist der Roboter 1 bzw. seine aktuelle Position x₀ durch eine virtuelle Feder steuerungstechnisch an eine Ankerposition xₛ gefesselt, die mit der aktuelle Position x₀ zur Grenze s hin verschiebbar ist. Zusätzlich kann der Roboter wie mit Bezug auf Fig. 2 erläutert durch einen virtuellen Dämpfer steuerungstechnisch inertial bzw. an die Umgebung gefesselt sein, was in Fig. 3 zur besseren Übersichtlichkeit nicht dargestellt ist.

Als Ankerposition xₛ wird jeweils eine Position innerhalb der kürzesten Verbindung zwischen der aktuellen Position x₀ und der Grenze s vorgegeben, die um eine vorgegebene Distanz δ von der aktuellen Position x₀ zu der Grenze s hin beabstandet ist. Hierdurch wird beständig eine im Wesentlichen konstante Rückstellkraft zur Grenze s hin aufgeprägt. Gleichermaßen kann auch die aktuelle Position des Roboters selber vorgegeben werden, um keine Rückstellkraft zur Grenze aufzuprägen, insbesondere wenigstens anfänglich nach einer Aktivierung der Grenzüberwachung.

Wie in Fig. 3 durch eine unidirektional sperrende Klinke symbolisch angedeutet, kann die Ankerposition xₛ durch eine externe Kraftbeaufschlagung, insbesondere Handführung, des Roboters 1 nicht von der Grenze s weg (nach rechts in Fig. 3) verschoben werden. Einer entsprechenden Bewegung des Roboters wirkt die virtuelle Feder entgegen, die sich zwischen der festgehaltenen Ankerposition xₛ und der sich von dieser entfernenden aktuellen Position x₀ spannt: T = c·(x₀-xₛ).

Die Ankerposition xₛ kann jedoch durch eine Bewegung des Roboters 1 bzw. seiner aktuellen Position x₀ zur Grenze hin (nach links in Fig. 3), sozusagen mit der aktuellen Position x₀ mit, verschoben werden.

Mit anderen Worten wird die Ankerposition xₛ mit bzw. entsprechend der aktuellen Position x₀ aktualisiert, sofern diese zur Grenze s hin verschoben wird, und nicht aktualisiert, sofern diese von der Grenze s weg verschoben wird.

Dadurch wird die steuerungstechnisch durch die virtuelle Feder aufgeprägte Rückstellkraft T, die den Roboter aus seiner aktuellen Position x₀ in dem gesperrten Bereich S zur Grenze s dieses Bereichs zurücktreibt, abhängig von einem Abstand der Position x₀ zu der mit ihr mitführbaren Ankerposition xₛ und somit von einem Abstand der Position x₀ zu der Grenze s selber unabhängig vorgegeben, sofern der Roboter um einen Weg zu der Grenze hin oder parallel zu der Grenze hin bewegt wird, insbesondere gleich Null, sofern als Ankerposition die aktuelle Position selber vorgegeben wird.

Auf der anderen Seite wird durch das Festhalten der Ankerposition xₛ eine andere, größere Rückstellkraft T steuerungstechnisch durch die virtuelle Feder aufgeprägt, sofern der Roboter um denselben Weg von der Grenze weg bewegt wird.

Diese Ausführung kann insbesondere anstelle der mit Bezug auf Fig. 2 erläuterten Ausführung mit der mit Bezug auf Fig. 1 erläuterten Ausführung kombiniert sein, d.h. die Rückstellkraft kann jeweils die erste (gegebenenfalls andere erste) Rückstellkraft T₁ sein (vgl. S40), die steuerungstechnisch kommandiert bzw. aufgeprägt wird, sofern der Roboter sich bereits bei Aktivierung der Grenzüberwachung in dem gesperrten Bereich S befindet (S30: "Y"). Tritt der Roboter hingegen erst nach Aktivierung der Grenzüberwachung in den gesperrten Bereich S ein, kann die zweite Rückstellkraft T₂ insbesondere wie in der eingangs genannten US 2004/0128026 A1 vorgegeben werden. Gleichermaßen kann die mit Bezug auf Fig. 3 erläuterte Vorgabe bzw. Aufprägung der Rückstellkraft auch unabhängig davon stets in der Grenzüberwachung durchgeführt werden, d.h. auch bei einem Eintreten des Roboters in den gesperrten Bereich erst nach Aktivierung der Grenzüberwachung (vgl. S70).

Hierdurch wird ebenfalls vorteilhaft eine überraschende massive Rückstellbewegung des Roboters 1 infolge einer Aktivierung der Grenzüberwachung in einer aktuellen Position im gesperrten Bereich verhindert.

Wie vorstehend erläutert, kann in einer Ausführung steuerungstechnisch zusätzlich eine Dämpfungskraft F aufgeprägt werden, die einer Bewegung des Roboters entgegenwirkt und von einer aktuellen Geschwindigkeit des Roboters abhängt, wie dies in Fig. 2 durch ein inertial gefesseltes Dämpfersymbol angedeutet ist und gleichermaßen auch bei der Ausführung der Fig. 3 der Fall sein kann.

Diese Dämpfungskraft F kann in einem einfachen Beispiel proportional zur aktuellen Geschwindigkeit dx₀/dt des Roboters sein: F = d·dx₀/dt.

In einer Ausführung ist der Proportionalitäts- bzw. Dämpfungsfaktor d unterhalb einer Mindestgeschwindigkeit gleich Null, während er über der Mindestgeschwindigkeit größer als Null ist, beispielsweise konstant oder mit einem Abstand zur Grenze s veränderlich. Damit steigt die Dämpfungskraft F in einem zweiten Geschwindigkeitsbereich oberhalb der vorgegebenen Mindestgeschwindigkeit aufgrund des Proportionalitäts- bzw. Dämpfungsfaktors d > 0 stärker mit der aktuellen Geschwindigkeit dx₀/dt des Roboters an als in einem ersten Geschwindigkeitsbereich unterhalb der Mindestgeschwindigkeit, in dem die Dämpfungskraft F aufgrund des Proportionalitäts- bzw. Dämpfungsfaktors d = 0 nicht mit der aktuellen Geschwindigkeit ansteigt.

Hierdurch ist es möglich, den Roboter unterhalb der Mindestgeschwindigkeit steuerungstechnisch schwächer, insbesondere im Wesentlichen ungedämpft, handzuführen, während er oberhalb der Mindestgeschwindigkeit überporoprtional stärker gedämpft wird. Auf diese Weise kann nicht nur ein Eindringen in einen gesperrten Bereich reduziert, sondern zusätzlich auch eine massive Rückstellbewegung bei Aktivierung der Grenzüberwachung verhindert oder jedenfalls reduziert werden.

Obwohl in der vorhergehenden Beschreibung exemplarische Ausführungen erläutert wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendungen und den Aufbau in keiner Weise einschränken sollen. Vielmehr wird dem Fachmann durch die vorausgehende Beschreibung ein Leitfaden für die Umsetzung von mindestens einer exemplarischen Ausführung gegeben, wobei diverse Änderungen, insbesondere in Hinblick auf die Funktion und Anordnung der beschriebenen Bestandteile, vorgenommen werden können, ohne den Schutzbereich zu verlassen, wie er sich aus den Ansprüchen ergibt.

### Bezugszeichenliste

- 1: Roboter
- 2: Steuerung
- x₍₀₎: (aktuelle) Position
- x_{S}: Ankerposition
- c_{1; 2}: (virtuelle) Federsteifigkeit
- d: Dämpfung(sfaktor)
- s: Grenze
- S: gesperrter Bereich x>s
- T_{(1, 2, 3)}: (Rückstell)Kraft
- F: Dämpfungskraft
- t: Zeit
- δ: Distanz

## Patentansprüche

1. Verfahren zum Steuern eines nachgiebig geregelten Roboters (1), mit dem Schritt:
Durchführen einer Grenzüberwachung des Roboters;
wobei eine erste Rückstellkraft (T₁), die den Roboter aus einer aktuellen Position (x₀) in einem gesperrten Bereich (S: x>s) zu einer Grenze (s) dieses Bereichs zurücktreibt, steuerungstechnisch vorgegeben wird (S40), sofern der Roboter sich bei Aktivierung der Grenzüberwachung bereits in dieser Position in dem gesperrten Bereich befindet, und
eine zweite Rückstellkraft (T₂), die den Roboter aus der Position (x₀) zu der Grenze (s) zurücktreibt, steuerungstechnisch vorgegeben wird (S70), sofern der Roboter erst nach Aktivierung der Grenzüberwachung diese Position in dem gesperrten Bereich einnimmt,
wobei die erste Rückstellkraft wenigstens zeitweise kleiner als die zweite Rückstellkraft, insbesondere gleich Null, ist.

2. Verfahren nach Anspruch 1, **dadurch gekennzeichnet, dass** eine Steifigkeit einer virtuellen Feder, die den Roboter steuerungstechnisch an eine Ankerposition (xₛ), insbesondere auf der Grenze, fesselt, vorgegeben wird (S40, S70), wobei eine erste Steifigkeit (c₁) vorgegeben wird (S40), sofern der Roboter sich bei Aktivierung der Grenzüberwachung bereits in einer Position (x₀) in dem gesperrten Bereich befindet, und eine zweite Steifigkeit (c₂) vorgegeben wird (S70), sofern der Roboter erst nach Aktivierung der Grenzüberwachung dieselbe Position (x₀) in dem gesperrten Bereich einnimmt, wobei die erste Steifigkeit wenigstens bei Aktivierung der Grenzüberwachung kleiner als die zweite Steifigkeit ist.

3. Verfahren nach einem der vorhergehenden Ansprüche, **dadurch gekennzeichnet, dass** eine Steifigkeit (c₁) einer virtuellen Feder, die den Roboter steuerungstechnisch an eine Ankerposition (xₛ), insbesondere auf der Grenze, fesselt, in Abhängigkeit von einem zeitlichen Abstand (t) zur Aktivierung der Grenzüberwachung vorgegeben wird (S40), insbesondere, sofern der Roboter sich bei Aktivierung der Grenzüberwachung bereits in dem gesperrten Bereich befindet.

4. Verfahren nach einem der vorhergehenden Ansprüche, **dadurch gekennzeichnet, dass** eine Steifigkeit (c₁) einer virtuellen Feder, die den Roboter steuerungstechnisch an eine Ankerposition (xₛ), insbesondere auf der Grenze, fesselt, in Abhängigkeit von einer Bewegung (d(x₀-s)/dt) des Roboters relativ zur Grenze vorgegeben wird, insbesondere bei einer Bewegung von der Grenze weg (d(x₀-s)/dt>0) größer als bei einer Bewegung (d(x₀-s)/dt<0) nicht von der Grenze weg, insbesondere, sofern der Roboter sich bei Aktivierung der Grenzüberwachung bereits in dem gesperrten Bereich befindet.

5. Verfahren zum Steuern eines nachgiebig geregelten Roboters nach einem der vorhergehenden Ansprüche, mit dem Schritt:
Durchführen einer Grenzüberwachung des Roboters;
wobei eine erste Rückstellkraft (T), die den Roboter aus einer aktuellen Position (x₀) in einem gesperrten Bereich (S: x>s) zu einer Grenze (s) dieses Bereichs zurücktreibt, von einem Abstand der Position zu der Grenze unabhängig, insbesondere gleich Null, steuerungstechnisch vorgegeben wird (S40), sofern der Roboter um einen Weg zu der Grenze hin oder parallel zu der Grenze hin bewegt wird (d(x₀-s)/dt<0), und eine andere Rückstellkraft (T), die größer als die erste Rückstellkraft ist, steuerungstechnisch vorgegeben wird (S40), sofern der Roboter um denselben Weg von der Grenze weg bewegt wird (d(x₀-s)/dt>0), insbesondere, sofern der Roboter sich bei Aktivierung der Grenzüberwachung bereits in dieser Position in dem gesperrten Bereich befindet.

6. Verfahren nach Anspruch 5, **dadurch gekennzeichnet, dass** eine Ankerposition (xₛ) einer virtuellen Feder, an die der Roboter steuerungstechnisch gefesselt ist, durch eine externe Kraftbeaufschlagung, insbesondere Handführung, des Roboters nicht von der Grenze weg verschiebbar ist.

7. Verfahren nach Anspruch 5 oder 6, **dadurch gekennzeichnet, dass** als eine Ankerposition (xₛ) einer virtuellen Feder, an die der Roboter steuerungstechnisch gefesselt ist, eine aktuelle Position (x₀) des Roboters oder eine Position innerhalb einer, insbesondere kürzesten, Verbindung zwischen der aktuellen Position (x₀) und der Grenze (s) vorgegeben wird, die um eine Distanz (δ) von der aktuellen Position (x₀) zu der Grenze (s) hin beabstandet ist.

8. Verfahren nach einem der vorhergehenden Ansprüche, **dadurch gekennzeichnet, dass** eine steuerungstechnisch aufgeprägte Dämpfungskraft (F), die einer Bewegung des Roboters entgegenwirkt und von einer aktuellen Geschwindigkeit (dx₀/dt) des Roboters abhängt, in einem zweiten Geschwindigkeitsbereich oberhalb einer vorgegebenen Mindestgeschwindigkeit stärker mit der aktuellen Geschwindigkeit des Roboters ansteigt als in einem ersten Geschwindigkeitsbereich unterhalb der Mindestgeschwindigkeit.

9. Steuerung (2) zum Steuern eines nachgiebig geregelten Roboters (1), die zur Durchführung eines Verfahrens nach einem der vorhergehenden Ansprüche eingerichtet ist.

10. Roboteranordnung mit einem mehrachsigen Roboter (1) und einer Steuerung (2) zum Steuern des nachgiebig geregelten Roboters nach einem der vorhergehenden Ansprüche.

11. Computerprogrammprodukt mit einem Programmcode, der auf einem von einem Computer lesbaren Medium gespeichert ist, zur Durchführung eines Verfahrens nach einem der vorhergehenden Ansprüche.

## Claims

1. A method of controlling a compliantly controlled robot (1), wherein the method comprises the step of:
carrying out a boundary monitoring of the robot;
wherein a first return force (T₁), which operates to return the robot back from a current position (x₀) in a blocked area (S: x>s) to a boundary (s) of this area, is specified (S40) by way of control technology, provided that the robot is already in this position in the blocked area upon activation of the boundary monitoring, and
wherein a second return force (T₂), which operates to return the robot back from the position (x₀) to the boundary (s), is specified by way of control technology (S70), provided that the robot assumes this position in the blocked area only after activation of the boundary monitoring,
wherein the first return force is at least temporarily smaller than the second return force, in particular equal to zero.

2. The method according to claim 1, **characterised in that** a stiffness of a virtual spring which, by way of control technology, ties the robot to an anchor position (xₛ), in particular on the boundary, is specified (S40, S70), wherein a first stiffness (c₁) is specified (S40), provided that the robot is already in a position (x₀) in the blocked area upon activation of the boundary monitoring, and a second stiffness (c₂) is specified (S70), provided that the robot assumes the same position (x₀) in the blocked area only after activation of the boundary monitoring, wherein, at least upon activation of the boundary monitoring, the first stiffness is smaller than the second stiffness.

3. The method according to any one of the preceding claims, **characterised in that** a stiffness (c₁) of a virtual spring which, by way of control technology, ties the robot to an anchor position (xₛ), in particular on the boundary, is specified (S40) as a function of a time lag (t) from the activation of the boundary monitoring, in particular if the robot is already located in the blocked area upon activation of the boundary monitoring.

4. The method according to any one of the preceding claims, **characterised in that** a stiffness (c₁) of a virtual spring, which, by way of control technology, ties the robot to an anchor position (xₛ), in particular on the boundary, is specified as a function of a movement (d(x₀-s)/dt) of the robot relative to the boundary, in particular that it is specified to be greater in the case of a movement away from the boundary (d(x₀-s)/dt>0) than in the case of a movement (d(x₀-s)/dt<0) not away from the boundary, in particular if the robot is already located in the blocked area upon activation of the boundary monitoring.

5. A method of controlling a compliantly controlled robot according to any one of the preceding claims, wherein the method comprises the step of:
carrying out a boundary monitoring of the robot;
wherein a first return force (T), which operates to return the robot from a current position (x₀) in a blocked area (S: x>s) to a boundary (s) of this area is specified (S40), by way of control technology, independently of a distance of the position to the boundary, in particular equal to zero, provided that the robot is moved a certain distance towards the boundary or parallel to the boundary (d(x₀-s)/dt<0), and wherein a different return force (T), which is greater than the first return force, is specified (S40), by way of control technology, provided that the robot is moved the same distance away from the boundary (d(x₀-s)/dt>0), in particular, if the robot is already in this position in the blocked area upon activation of the boundary monitoring.

6. The method according to claim 5, **characterised in that** an anchor position (xₛ) of a virtual spring, to which the robot is tied by way of control technology, cannot be displaced away from the boundary by an application of an external force, in particular by an application of a manual guidance force, to the robot.

7. The method according to claim 5 or 6, **characterised in that** a current position (x₀) of the robot or a position within a connection, in particular a shortest connection, between the current position (x₀) and the boundary (s), which is spaced apart, by a distance (δ), from the current position (x₀) towards the boundary (s), is specified as an anchor position (xₛ) of a virtual spring, to which the robot is tied by way of control technology.

8. The method according to any one of the preceding claims, **characterised in that** a damping force (F), which is applied by way of control technology and which counteracts a movement of the robot and which depends on a current speed (dx₀/dt) of the robot, increases more strongly, in dependence upon the current speed of the robot, in a second speed range above a predetermined minimum speed than in a first speed range below the minimum speed.

9. A control system (2) for controlling a compliantly controlled robot (1), which is set up to carry out a method according to any one of the preceding claims.

10. A robot assembly comprising a multi-axis robot (1) and a control system (2) for controlling the compliantly controlled robot according to any one of the preceding claims.

11. A computer program product with a program code which is stored on a computer-readable medium for carrying out a method according to any one of the preceding claims.

## Revendications

1. Procédé de commande d'un robot (1) réglé de façon élastique, avec l'étape de :
réalisation d'une surveillance des limites du robot ;
dans lequel une première force de rappel (T₁), qui fait reculer le robot d'une position (x₀) actuelle dans une zone bloquée (S : x>s) vers une limite (s) de cette zone, est prédéfinie par commande (S40), dans la mesure où le robot se trouve déjà lors de l'activation de la surveillance des limites dans cette position dans la zone bloquée, et
une deuxième force de rappel (T₂), qui fait reculer le robot de la position (x₀) vers la limite (s), est prédéfinie par commande (S70), dans la mesure où le robot n'adopte qu'après l'activation de la surveillance des limites cette position dans la zone bloquée,
dans lequel la première force de rappel est au moins temporairement inférieure à la deuxième force de rappel, en particulier égale à zéro.

2. Procédé selon la revendication 1, **caractérisé en ce qu'**une raideur d'un ressort virtuel, qui enchaîne le robot par commande à une position d'ancrage (xₛ), en particulier sur la limite, est prédéfinie (S40, S70), dans lequel une première raideur (c₁) est prédéfinie (S40), dans la mesure où le robot se trouve déjà lors de l'activation de la surveillance des limites dans une position (x₀) dans la zone bloquée, et une deuxième raideur (c₂) est prédéfinie (S70), dans la mesure où le robot n'adopte qu'après l'activation de la surveillance des limites cette position (x₀) dans la zone bloquée, dans lequel la première raideur au moins lors de l'activation de la surveillance des limites est inférieure à la deuxième raideur.

3. Procédé selon l'une quelconque des revendications précédentes, **caractérisé en ce qu'**une raideur (c₁) d'un ressort virtuel, qui enchaîne le robot par commande à une position d'ancrage (xₛ), en particulier sur la limite, est prédéfinie (S40) en fonction d'une distance temporelle (t) pour l'activation de la surveillance des limites, en particulier, dans la mesure où le robot se trouve déjà lors de l'activation de la surveillance des limites dans la zone bloquée.

4. Procédé selon l'une quelconque des revendications précédentes, **caractérisé en ce qu'**une raideur (c₁) d'un ressort virtuel, qui enchaîne le robot par commande à une position d'ancrage (xₛ), en particulier sur la limite, est prédéfinie en fonction d'un déplacement (d(x₀-s)/dt) du robot par rapport à la limite, en particulier lors d'un déplacement s'éloignant de la limite (d(x₀-s)/dt>0) plus grande que lors d'un déplacement (d(x₀-s)/dt<0) ne s'éloignant pas de la limite, en particulier, dans la mesure où le robot se trouve déjà lors de l'activation de la surveillance des limites dans la zone bloquée.

5. Procédé de commande d'un robot réglé de façon élastique selon l'une quelconque des revendications précédentes, avec l'étape de :
réalisation d'une surveillance des limites du robot ;
dans lequel une première force de rappel (T), qui fait reculer le robot d'une position (x₀) actuelle dans une zone bloquée (S : x>s) vers une limite (s) de cette zone, est prédéfinie par commande (S40), indépendamment d'une distance de la position par rapport à la limite, en particulier égale à zéro, dans la mesure où le robot est déplacé d'un trajet vers la limite ou parallèlement à la limite (d(x₀-s)/dt<0), et une autre force de rappel (T), qui est supérieure à la première force de rappel, est prédéfinie par commande (S40), dans la mesure où le robot est déplacé du même trajet en s'éloignant de limite (d(x₀-s)/dt>0), en particulier, dans le mesure où le robot se trouve déjà lors de l'activation de la surveillance des limites dans cette position dans la zone bloquée.

6. Procédé selon la revendication 5, **caractérisé en ce qu'**une position d'ancrage (xₛ) d'un ressort virtuel, à laquelle le robot est enchaîné par commande, ne peut pas être déplacée en s'éloignant de la limite par une sollicitation de force externe, en particulier un guidage manuel, du robot.

7. Procédé selon la revendication 5 ou 6, **caractérisé en ce qu'**une position (x₀) actuelle du robot ou une position à l'intérieur d'une liaison, en particulier la plus courte, entre la position (x₀) actuelle et la limite (s) est prédéfinie en tant que position d'ancrage (xₛ) d'un ressort virtuel, qui est éloignée d'une distance (δ) de la position (x₀) actuelle par rapport à la limite (s).

8. Procédé selon l'une quelconque des revendications précédentes, **caractérisé en ce qu'**une force d'amortissement (F) appliquée par commande, qui contrecarre un déplacement du robot et dépend d'une vitesse actuelle (dx₀/dt) du robot, augmente dans une deuxième plage de vitesse au-dessus d'une vitesse minimum prédéfinie davantage avec la vitesse actuelle du robot que dans une première plage de vitesse inférieure à la vitesse minimum.

9. Commande (2) pour la commande d'un robot (1) réglé de façon élastique, qui est aménagée pour la réalisation d'un procédé selon l'une quelconque des revendications précédentes.

10. Agencement de robot avec un robot (1) multiaxe et une commande (2) pour la commande d'un robot réglé de façon élastique, selon l'une quelconque des revendications précédentes.

11. Produit de programme informatique avec un code de programme, qui est stocké sur un support lisible par un ordinateur, pour la réalisation d'un procédé selon l'une quelconque des revendications précédentes.
